# EUROPEAN PATENT APPLICATION

(11) **EP 3 950 859 A1**
(43) Date of publication of application: **09.02.2022**
(21) Application number: 20781763.6
(22) Date of filing: 31.03.2020
(51) Int. Cl.: C09D 17/00, C09D 11/328, D06P 1/16, D06P 1/20, D06P 5/26, C09B 1/50, C09B 1/503, C09B 1/54, C09B 67/20, C09B 67/46

(54) **COLORED DISPERSION, RECORDING MEDIUM, AND HYDROPHOBIC FIBER PRINTING METHOD**

(30) Priority: 04.04.2019 JP 2019071867
(71) Applicant: Nippon Kayaku Kabushiki Kaisha, Tokyo 100-0005 (JP)
(72) Inventor: HANAZATO Akitsu, Tokyo 115-8588 (JP); MIYAZAWA Yoshimasa, Tokyo 115-8588 (JP); YOSHIMOTO Takashi, Tokyo 115-8588 (JP); TERANISHI Makoto, Tokyo 115-8588 (JP)
(74) Representative: Parchmann, Stefanie
(86) International application number: PCT/JP2020/014874
(87) International publication number: WO 2020/204042

(57) **Abstract**

Provided is a colored dispersion that contains (A) a dye derivative, (B) a water-insoluble dye, (C) a dispersant, and water, wherein the (A) component contains an anthraquinone-based compound represented by formula (a1), the (C) component contains a polyoxyethylene aryl phenyl ether and a polyoxyethylene aryl phenyl ether sulfate, and the mass ratio ((B)/(A)) of the (B) component with respect to the (A) component satisfies the relationship 400 > (B)/(A) > 3.125. Also provided are: a recording medium having said colored dispersion adhered thereto and a hydrophobic fiber printing method employing the colored dispersion. In formula (a1), each of R^{a1}-R^{a5} is a hydrogen atom or the like, and X^{a1} is a group represented by formula (a4) or the like. In formula (a4), Z^{a3} is an optionally-substituted amino group or the like.

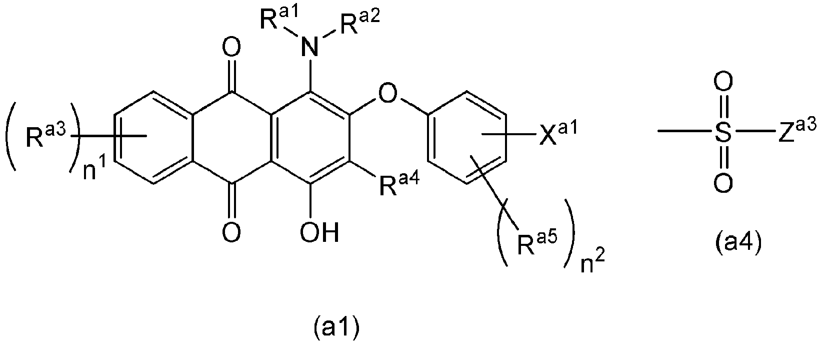

## Description

### TECHNICAL FIELD

The present invention relates to a colored dispersion liquid including an anthraquinone-based compound, a recording medium to which the colored dispersion liquid is adhered, and a method for textile printing of a hydrophobic fiber using the colored dispersion liquid.

### BACKGROUND ART

In recent years, there has been proposed a recording method for performing plate-less printing by inkjet, and textile printing by inkjet printing (inkjet textile printing) has also been performed in the printing of fibers including cloth, etc. Textile printing by inkjet printing has various advantages such as plate-free, resource saving, energy saving, and easy high-definition expression, compared to conventional textile printing methods such as screen printing, etc.

Here, hydrophobic fibers typified by polyester fibers are generally dyed by a water-insoluble coloring material. For this reason, as an aqueous ink for printing of hydrophobic fibers by inkjet printing, it is generally necessary to use dispersed inks, in which a water-insoluble coloring matter is dispersed in water, and which have good performance such as dispersion stability.

Inkjet textile printing methods for hydrophobic fibers are roughly divided into direct printing and sublimation transfer methods. The direct printing method is a textile printing method in which ink is directly applied (printed) to a hydrophobic fiber, and then a dye in the ink is heat treated by high temperature steaming or the like, so that the dye is dyed and fixed to the hydrophobic fiber. On the other hand, the sublimation transfer method is a textile printing method in which ink is applied (printed) to an intermediate recording medium (a special transfer paper, etc.), then an ink application surface of the intermediate recording medium and a hydrophobic fiber are superimposed on each other, and then a dye is transferred by heat from the intermediate recording medium to the hydrophobic fiber.

The sublimation transfer method is mainly used for printing of banner flags, etc. and easily sublimating dyes which are excellent in transferability to hydrophobic fibers by heat treatment are used in the ink. The processing steps include the two steps of (1) a printing step: a step of applying a dye ink to an intermediate recording medium by an inkjet printer; and (2) a transfer step: a step of transferring and dyeing the dye from the intermediate recording medium to the fiber by heat treatment. Since commercially available transfer papers can be widely used, pretreatment of fibers is not required, and a washing step is also omitted.

As an ink for use in the sublimation transfer method, a waterborne ink in which a water-insoluble dye is dispersed in water has been generally used. For example, Patent Document 1 describes that an aqueous ink is prepared by adding a water-soluble organic solvent as a moisturizing agent (drying inhibitor), a surfactant as a surface tension adjusting agent, and other additives (a pH adjuster, a preservative antifungal agent, a defoaming agent, and the like) to a dye dispersion liquid obtained by dispersing a water-insoluble dye selected from a disperse dye and an oil-soluble dye in water using a dispersant, to optimize physical characteristics (physical properties) such as particle size, viscosity, surface tension, pH, etc.

Patent Document 1: PCT International Publication No. WO2005/121263

### DISCLOSURE OF THE INVENTION

### Problems to be Solved by the Invention

However, when the present inventors have studied a conventional aqueous ink in which a water-insoluble dye is dispersed in water, it has been found that, even when the dispersion stability of the dye dispersion is relatively good, when a component such as a surfactant is added to the dye dispersion to prepare an aqueous ink, the particles in the aqueous ink aggregate and the dispersion stability decreases.

It is an object of the present invention to provide a colored dispersion liquid which is excellent in dispersion stability and suppresses aggregation of particles during storage, a recording medium to which the colored dispersion liquid is adhered, and a method for textile printing of a hydrophobic fiber using the colored dispersion liquid.

### Means for Solving the Problems

Specific means for solving the above problem include the following embodiments.

A first aspect of the present invention relates to a colored dispersion liquid, containing: (A) a dye derivative, (B) a water-insoluble dye, (C) a dispersant, and water, in which the dye derivative (A) includes an anthraquinone-based compound represented by the following formula (a1): 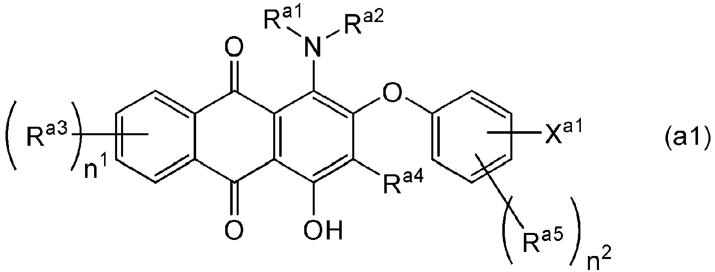
in the formula, R^{a1} to R^{a5} each independently represent a hydrogen atom, a C1 to C4 alkyl group which may have a substituent, a cyclic alkyl group which may have a substituent, an aryl group which may have a substituent, or a halogen atom, n¹ and n² each independently represent an integer of 1 to 4, when n¹ represents an integer of 2 to 4, R^{a3} in the number of n¹ may be the same as or different from each other, when n² represents an integer of 2 to 4, R^{a5} in the number of n² may be the same as or different from each other, and X^{a1} represents a group represented by any one of the following formulas (a2) to (a5): 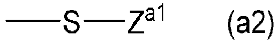 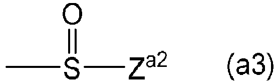 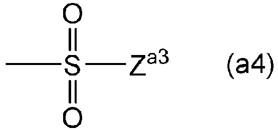 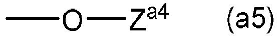
in the formulas, Z^{a1} represents an aliphatic hydrocarbon group which may have a substituent or an aromatic hydrocarbon group which may have a substituent, Z^{a2} represents an amino group which may have a substituent or an alkoxy group which may have a substituent, Z^{a3} represents an amino group which may have a substituent, an aliphatic hydrocarbon group which may have a substituent, an aromatic hydrocarbon group which may have a substituent, an alkoxy group which may have a substituent, an aryloxy group which may have a substituent, a pyridinooxy group, a hydroxy group, or a halogen atom, and Z^{a4} represents a C1 to C4 alkyl group,
in which the dye derivative (A) does not simultaneously include a compound represented by the formula (a1) in which X^{a1} represents a group represented by the formula (a4) and a compound represented by the formula (a1) in which X^{a1} represents a group represented by the formula (a5),
in which the dispersant (C) includes a polyoxyethylene arylphenyl ether and a polyoxyethylene arylphenyl ether sulfate, and
in which a mass ratio ((B)/(A)) of the water-insoluble dye (B) to the dye derivative (A) satisfies a relation of 400 > (B)/(A) > 3.125.

A second aspect of the present invention relates to the colored dispersion liquid as described in the first aspect, in which the mass ratio ((B)/(A)) of the water-insoluble dye (B) to the dye derivative (A) satisfies a relation of 400 > (B)/(A) > 19.

A third aspect of the present invention relates to the colored dispersion liquid as described in the first or second aspect, in which the anthraquinone-based compound represented by the formula (a1) includes a compound represented by the following formula (a1-1): 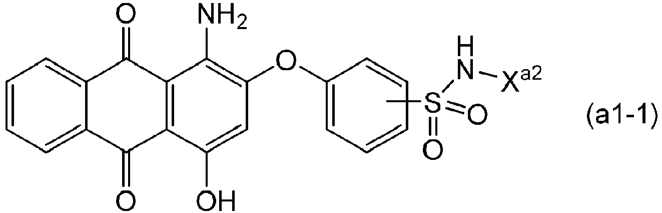 in the formula, X^{a2} represents a hydrogen atom, a phenyl group, a n-butyl group, or a 3-ethoxypropyl group.

A fourth aspect of the present invention relates to the colored dispersion liquid as described in any one of the first to third aspects, in which the water-insoluble dye (B) is a disperse dye.

A fifth aspect of the present invention relates to the colored dispersion liquid as described in the fourth aspect, in which the disperse dye is a disperse dye having an anthraquinone skeleton.

A sixth aspect of the present invention relates to the colored dispersion liquid as described in the fifth aspect, in which the disperse dye having an anthraquinone skeleton is a disperse dye represented by the following formula (b1): 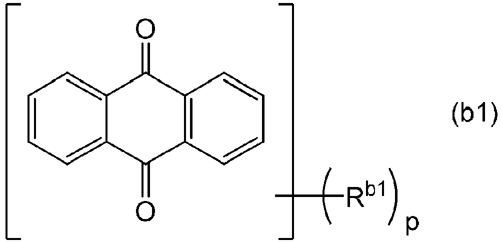 in the formula, R^{b1} represents a hydrogen atom or a substituent, p represents an integer of 0 to 6, and when p represents an integer of 2 to 6, R^{b1} in the number of p may be the same as or different from each other.

A seventh as aspect of the present invention relates to the colored dispersion liquid as described in the sixth aspect, in which the disperse dye represented by the formula (b1) is C.I. Disperse Red 60.

An eighth aspect of the present invention relates to a recording medium, including the colored dispersion liquid as described in any one of the first to seventh aspects adhered thereto.

A ninth aspect of the present invention relates to the recording medium as described in the eighth aspect, in which the recording medium is a hydrophobic fiber.

A tenth aspect of the present invention relates to a method for textile printing of a hydrophobic fiber, including: printing by adhering a droplet of the colored dispersion liquid as described in any one of the first to seventh aspects to an intermediate recording medium to obtain a recorded image, and
transferring the recorded image to the hydrophobic fiber by contacting a hydrophobic fiber with a surface of the intermediate recording medium on which the colored dispersion liquid is adhered, followed by heat treatment.

### Effects of the Invention

According to the present invention, it is possible to provide a colored dispersion liquid which is excellent in dispersion stability and in which aggregation of particles during storage is suppressed; a recording medium to which the colored dispersion liquid is adhered; and a method for textile printing of a hydrophobic fiber using the colored dispersion liquid.

### PREFERRED MODE FOR CARRYING OUT THE INVENTION

### <Colored dispersion liquid>

The colored dispersion liquid as described in the present embodiment contains (A) a dye derivative (hereinafter, also referred to as "component (A)"), (B) a water-insoluble dye (hereinafter, also referred to as "component (B)"), (C) a dispersant (hereinafter, also referred to as "component"(C))), and water. Hereinafter, the respective components contained in the colored dispersion liquid as described in the present embodiment will be described.

### [Dye derivative (A)]

The colored dispersion liquid as described in the present embodiment contains an anthraquinone-based compound represented by the following formula (a1), as the dye derivative (A). When the colored dispersion liquid contains the anthraquinone-based compound represented by the following formula (a1), the dispersed state of particles in the colored dispersion liquid tends to be stabilized.

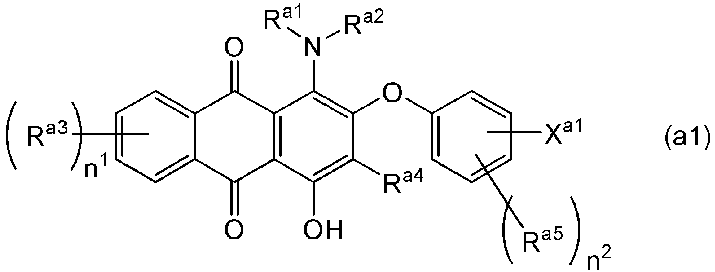

In the formula (a1), R^{a1} to R^{a5} each independently represent a hydrogen atom, a C1 to C4 alkyl group which may have a substituent, a cyclic alkyl group which may have a substituent, an aryl group which may have a substituent, or a halogen atom, n¹ and n² each independently represent an integer of 1 to 4, when n¹ represents an integer of 2 to 4, R^{a3} in the number of n¹ may be the same as or different from each other, and when n² represents an integer of 2 to 4, R^{a5} in the number of n² may be the same as or different from each other.

The C1 to C4 alkyl group may be linear or branched, and is preferably linear. Examples of the C1 to C4 alkyl group include a methyl group, an ethyl group, a n-propyl group, an isopropyl group, a n-butyl group, a sec-butyl group, a tert-butyl group, etc., and a methyl group or an ethyl group is preferred. Examples of the cyclic alkyl group include a cyclohexyl group, a cyclopentyl group, etc. Examples of the aryl group include a phenyl group, a naphthyl group, etc. Examples of the halogen atom include a fluorine atom, a chlorine atom, a bromine atom, an iodine atom, etc.

The substituent which the C1 to C4 alkyl group, the cyclic alkyl group, or the aryl group may have is not particularly limited. Examples of the substituent include at least one selected from the group consisting of a halogen atom, a cyano group, a hydroxy group, an amino group, a C1 to C4 alkoxy group, and an aryloxy group. Examples of the halogen atom include the same halogen atoms as those described above. Examples of the C1 to C4 alkoxy group include a methoxy group, an ethoxy group, a n-butoxy group, etc. Examples of the aryloxy group include a phenoxy group, a naphthoxy group, etc.

In the formula (a1), X^{a1} represents a group represented by any one of the following formulas (a2) to (a5).

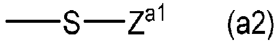

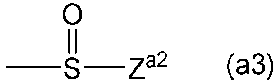

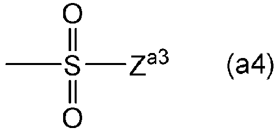

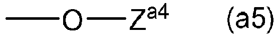

In the above formulas, Z^{a1} represents an aliphatic hydrocarbon group which may have a substituent or an aromatic hydrocarbon group which may have a substituent. Z^{a2} represents an amino group which may have a substituent or an alkoxy group which may have a substituent. Z^{a3} represents an amino group which may have a substituent, an aliphatic hydrocarbon group which may have a substituent, an aromatic hydrocarbon group which may have a substituent, an alkoxy group which may have a substituent, an aryloxy group which may have a substituent, a pyridinooxy group, a hydroxy group, or a halogen atom. Z^{a4} represents a C1 to C4 alkyl group.

Examples of the aliphatic hydrocarbon group include a linear or branched C1 to C8 alkyl group, a cyclic alkyl group, etc. Examples of the C1 to C8 alkyl group include a methyl group, an ethyl group, a n-propyl group, an isopropyl group, a n-butyl group, a sec-butyl group, a tert-butyl group, a n-hexyl group, a n-octyl group, etc. Examples of the cyclic alkyl group include a cyclobutyl group, a cyclohexyl group, a cyclopentyl group, etc. The cyclic alkyl group may include a hetero atom as the ring component.

Examples of the aromatic hydrocarbon group include a phenyl group, a naphthyl group, a fluorenyl group, an anthracenyl group, etc.

Examples of the alkoxy group, the aryloxy group, and the halogen atom include the same groups as those described above.

The substituent which the aliphatic hydrocarbon group, the aromatic hydrocarbon group, the amino group, the alkoxy group, or the aryloxy group may have is not particularly limited. Examples of the substituent include at least one selected from the group consisting of a halogen atom, a cyano group, a hydroxy group, an amino group, an aliphatic hydrocarbon group, an aromatic hydrocarbon group, a heterocyclic group, a C1 to C4 alkoxy group, and an aryloxy group. Examples of the heterocyclic group include a pyrrole ring group, a benzothiazole ring group, etc. Examples of the halogen atom, the aliphatic hydrocarbon group, the aromatic hydrocarbon group, the C1 to C4 alkoxy group, and the aryloxy group include the same groups as those described above.

Among the groups represented by the above formulas (a2) to (a5), groups represented by the above formulas (a2) to (a4) are preferred, and a group represented by the above formula (a4) is more preferred.

Examples of preferred compounds among the anthraquinone-based compounds represented by the formula (a1) include compounds represented by the following formula (a1-1).

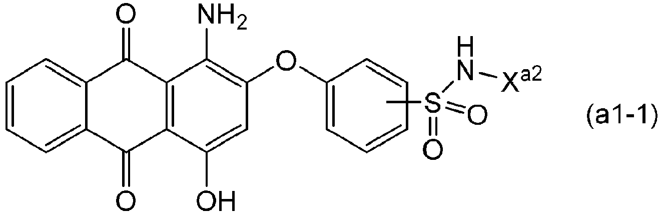

In the formula (a1-1), X^{a2} represents a hydrogen atom, a phenyl group, a n-butyl group, or a 3-ethoxypropyl group.

With regard to the anthraquinone-based compounds represented by the formula (a1), one type may be used alone, or two or more types thereof may be used in combination. However, the colored dispersion liquid as described in the present embodiment does not contain simultaneously a compound of the formula (a1) in which X^{a1} represents a group represented by the formula (a4), and a compound of the formula (a1) in which X^{a1} represents a group represented by the formula (a5).

Note that when the dye derivative (A) includes a compound of the formula (a1-1) in which X^{a2} represents a 3-ethoxypropyl group, the water-insoluble dye (B) to be described below includes neither Disperse Yellow 114 nor an azo compound having a benzothiazole skeleton.

The anthraquinone-based compound represented by the formula (a1) can be obtained, for example, by reacting C.I. Disperse Red 60 in chlorosulfonic acid, or by chlorinating a sulfonate of a dye in an organic solvent using a chlorinating agent such as thionyl chloride, oxalyl chloride, or phosphorus trichloride, or the like to obtain a chlorosulfonated dye, followed by reacting the chlorosulfonated dye with a desired amine.

The content of the dye derivative (A) is preferably from 0.0025 to 10% by mass, and more preferably from 0.025 to 5% by mass, with respect to the total amount of the colored dispersion liquid, from the viewpoint of securing a degree of freedom of the composition during preparation of the colored dispersion liquid and the stability of the colored dispersion liquid.

### [Water-insoluble dye (B)]

The water-insoluble dye (B) is not particularly limited as long as it is a dye insoluble or sparingly soluble in water. Examples of such dyes include disperse dyes, oil-soluble dyes, and the like, and disperse dyes are preferred. Note that "water-insoluble" in this specification means that the solubility in water at 25°C is not more than 1 g/m³.

Examples of disperse dyes include: C.I. Disperse Yellow 3, 4, 5, 7, 9, 13, 24, 30, 33, 34, 42, 44, 49, 50, 51, 54, 56, 58, 60, 63, 64, 66, 68, 71, 74, 76, 79, 82, 83, 85, 86, 88, 90, 91, 93, 98, 99, 100, 104, 114, 116, 118, 119, 122, 124, 126, 135, 140, 141, 149, 160, 162, 163, 164, 165, 179, 180, 182, 183, 184, 186, 192, 198, 199, 201, 202, 204, 210, 211, 215, 216, 218, 224, 231, 232, and 241; C.I. Disperse Orange 1, 3, 5, 7, 11, 13, 17, 20, 21, 25, 29, 30, 31, 32, 33, 37, 38, 42, 43, 44, 45, 47, 48, 49, 50, 53, 54, 55, 56, 57, 58, 59, 61, 66, 71, 73, 76, 78, 80, 89, 90, 91, 93, 96, 97, 119, 127, 130, 139, and 142; C.I. Disperse Red 1, 4, 5, 7, 11, 12, 13, 15, 17, 27, 43, 44, 50, 52, 53, 54, 55, 56, 58, 59, 60, 65, 72, 73, 74, 75, 76, 78, 81, 82, 86, 88, 90, 91, 93, 96, 103, 105, 106, 107, 108, 110, 111, 113, 117, 118, 121, 122, 126, 127, 128, 131, 132, 134, 135, 137, 143, 145, 146, 151, 152, 153, 154, 157, 159, 164, 167, 169, 177, 179, 181, 183, 184, 185, 188, 189, 190, 191, 192, 200, 201, 202, 203, 205, 206, 207, 210, 221, 224, 225, 227, 229, 239, 240, 257, 258, 277, 278, 279, 281, 288, 289, 298, 302, 303, 310, 311, 312, 320, 324, 328, 343, 362, and 364; C.I. Disperse Violet 1, 4, 8, 23, 26, 27, 28, 31, 33, 35, 36, 38, 40, 43, 46, 48, 50, 51, 52, 56, 57, 59, 61, 63, 69, and 77; C.I. Disperse Green 6:1, and 9; C.I. Disperse Brown 1, 2, 4, 9, 13, 19, and 27; C.I. Disperse Blue 3, 7, 9, 14, 16, 19, 20, 26, 27, 35, 43, 44, 54, 55, 56, 58, 60, 62, 64, 71, 72, 73, 75, 79, 81, 82, 83, 87, 91, 93, 94, 95, 96, 102, 106, 108, 112, 113, 115, 118, 120, 122, 125, 128, 130, 139, 141, 142, 143, 146, 148, 149, 153, 154, 158, 165, 167, 171, 173, 174, 176, 181, 183, 185, 186, 187, 189, 197, 198, 200, 201, 205, 207, 211, 214, 224, 225, 257, 259, 267, 268, 270, 284, 285, 287, 288, 291, 293, 295, 297, 301, 315, 330, 333, 343, 359, and 360; and C.I. Disperse Black 1, 3, 10, and 24. Of these disperse dyes, one type may be used alone or two or more types thereof may be used in combination.

As the water-insoluble dye (B), a disperse dye having an anthraquinone skeleton is preferred among the above-mentioned disperse dyes, and a disperse dye represented by the following formula (b1) is more preferred.

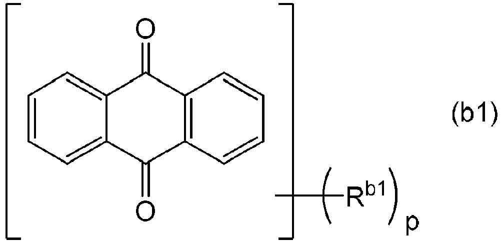

In the formula (b1), R^{b1} represents a hydrogen atom or a substituent. p represents an integer between 0 and 6. When p represents an integer between 2 and 6, R^{b1} in the number of p may be the same as or different from each other.

The substituent is not particularly limited. Examples of the substituent include at least one selected from the group consisting of a hydroxy group, an amino group, and an aryloxy group which may have a substituent. Examples of the aryloxy group which may have a substituent include the same groups as those described above.

As a preferred disperse dye among the disperse dyes represented by the formula (b1), C.I. Disperse Red 60 can be mentioned.

The average particle diameter of the water-insoluble dye (B) is appropriately decided depending on the application. For example, when the colored dispersion liquid as described in the present embodiment is used for inkjet printing, the average particle diameter of the water-insoluble dye (B) is preferably 50 to 200 nm, from the viewpoint of discharge properties. Note that the average particle diameter can be measured by a general method such as a dynamic light scattering method, a laser diffraction method, etc.

The content (solid content) of the water-insoluble dye (B) is preferably 0.1 to 25% by mass, and more preferably 0.5 to 20% by mass, with respect to the total amount of the colored dispersion liquid, from the viewpoint of securing the degree of freedom of the composition and the coloring performance at the time of preparation of the colored dispersion liquid.

In particular, the colored dispersion liquid as described in the present embodiment satisfies relation in which the mass ratio ((B)/(A)) of the water-insoluble dye (B) to the dye derivative (A) satisfies a relation of 400 > (B)/(A) > 3.125. When the ratio (B)/(A) satisfies the above relation, the dispersion state of particles in the colored dispersion liquid tends to be stable. The ratio (B)/(A) preferably satisfies a relation of 400 > (B)/(A) > 3.34, more preferably satisfies a relation of 400 > (B)/(A) > 19, more preferably satisfies a relation of 200 ≥ (B)/(A) > 19, particularly preferably satisfies a relation of 100 ≥ (B)/(A) ≥ 20, and extremely preferably satisfies a relation of 100 ≥ (B)/(A) > 20.

### [Dispersant (C)]

As the dispersant (C), the colored dispersion liquid as described in the present embodiment contains polyoxyethylene arylphenyl ethers and polyoxyethylene arylphenyl ether sulfates as the dispersant (C).

Examples of the polyoxyethylene arylphenyl ethers include styrylphenol compounds such as polyoxyethylene monostyrylphenyl ether, polyoxyethylene distyrylphenyl ether, polyoxyethylene tristyrylphenyl ether, polyoxyethylene tetrastyrylphenyl ether etc.; benzylphenol compounds such as polyoxyethylene monobenzylphenyl ether, polyoxyethylene dibenzylphenyl ether, polyoxyethylene tribenzylphenyl ether, etc.; cumylphenol compounds such as polyoxyethylene cumylphenyl ether etc.; polyoxyethylene naphthyl phenyl ether, polyoxyethylene biphenyl ether, and polyoxyethylene phenoxyphenyl ether; and the like. Among these, polyoxyethylene distyrylphenyl ether, polyoxyethylene tristyrylphenyl ether, polyoxyethylene dibenzylphenyl ether, polyoxyethylene tribenzylphenyl ether, and polyoxyethylene cumylphenyl ether are preferred. With respect to the polyoxyethylene arylphenyl ethers, one type may be used alone or two or more types may be used in combination.

The repeating number of the polyoxyethylene group in the polyoxyethylene arylphenyl ether is preferably 1 to 30, and more preferably 15 to 30. When the repeating number is 1 or more, compatibility with an aqueous solvent or the like tends to be excellent. Further, when the repeating number is 30 or less, the viscosity tends not to be too high.

As a commercial product of the polyoxyethylene arylphenyl ether, the following can be mentioned: Noigen EA series (manufactured by DKS Co., Ltd.); Pionin D-6112, Pionin D-6115, Pionin D-6120, Pionin D-6131, Pionin D-6512, Takesurf D-6413, DTD-51, Pionin D-6112, and Pionin D-6320 (all of which are manufactured by Takemoto Oil & Fat Co., Ltd.); TS-1500, TS-2000, TS-2600, SM-174N (all of which are manufactured by Toho Chemical Industry Co., Ltd.); Emulgen A60, Emulgen A90, and Emulgen A500 (all of which are manufactured by Kao Corporation); Emulgen B-66, Newcol CMP series (all of which are manufactured by Nippon Nyukazai Co., Ltd.); and the like.

Examples of the polyoxyethylene arylphenyl ether sulfate include sulfate salts of the above-described polyoxyethylene arylphenyl ethers. With respect to the polyoxyethylene arylphenyl ether sulfates, one type may be used alone or two or more types may be used in combination.

Examples of commercially available products of polyoxyethylene arylphenyl ether sulfates include SM-57, SM-130, SM-210 (all of which are manufactured by Toho Chemical Industry Co., Ltd.) and the like.

The content of the dispersant (C) is preferably 1 to 300% by mass, more preferably 5 to 120% by mass, with respect to the total amount of the water-insoluble dye (B).

### [Water]

As the water, that having few impurities such as ion-exchanged water, distilled water, ultrapure water, etc. are preferred. The content of water in the colored dispersion liquid is appropriately selected depending on the application. The content of water in the colored dispersion liquid is usually 200 to 8,500 parts by mass per 100 parts by mass of the water-insoluble dye (B).

### [Additives]

The colored dispersion liquid as described in the present embodiment may include additives other than the above. Examples of the additives include a water-soluble organic solvent, a preservative, a surfactant, a pH adjuster, a chelating reagent, a rust inhibitor, a water-soluble ultraviolet absorber, a water-soluble polymer compound, a viscosity modifier, a dye dissolving agent, an antioxidant, a resin emulsion, etc. Among these, it is preferable for the colored dispersion liquid as described in the present embodiment to include at least one selected from the group consisting of a water-soluble organic solvent, a preservative, a surfactant, and a pH adjuster.

The content of the water-soluble organic solvent is preferably 0 to 90% by mass, more preferably 0.01 to 85% by mass, with respect to the total amount of the colored dispersion liquid. Further, the content of the total of the other additives is preferably 0 to 50% by mass, more preferably 0.01 to 10% by mass, with respective to the total amount of the colored dispersion liquid.

Examples of the water-soluble organic solvent include glycol-based solvents, polyhydric alcohols, pyrrolidones, etc. Examples of the glycol-based solvent include glycerin, polyglycerin (#310, #750, #800), diglycerin, triglycerin, tetraglycerin, pentaglycerin, hexaglycerin, heptaglycerin, octaglycerin, nonaglycerin, decaglycerin, undecaglycerin, dodecaglycerin, tridecaglycerin, tetradecaglycerin, etc. Examples of the polyhydric alcohols include C2 to C6 polyhydric alcohols having 2 to 3 alcoholic hydroxy groups; di or tri C2 to C3 alkylene glycols; poly C2 to C3 alkylene glycols having 4 or more repeating units and having a molecular weight of about 20,000 or less, and preferably include liquid polyalkylene glycols, and the like. Examples thereof include ethylene glycol, diethylene glycol, triethylene glycol, propylene glycol, polyethylene glycol, polypropylene glycol, 1,3-propanediol, 1,2-butanediol, thiodiglycol, 1,3-butanediol, 1,4-butanediol, 2,3-butanediol, 3-methyl-1,3-butanediol, 1,2-pentanediol, 1,5-pentanediol, 2-methyl-2,4-pentanediol, 3-methyl-1,5-pentanediol, 1,2-hexanediol, 1,6-hexanediol, glycerin, trimethylolpropane, 1,3-pentanediol, 1,5-pentanediol, and the like. Examples of the pyrrolidone include 2-pyrrolidone, N-methyl-2-pyrrolidone, etc. Further, a compound or the like which is dissolved in water and serves as a wetting agent is also included in the water-soluble organic solvent, for convenience sake. Examples of such a compound include urea, ethylene urea, saccharides, etc.

In view of the storage stability of the colored dispersion liquid as described in the present embodiment, as the water-soluble organic solvent, a solvent having a small solubility for the water-insoluble dye (B) is preferred. It is particularly preferred to use glycerin and a solvent other than glycerin (preferably a polyhydric alcohol other than glycerin) in combination.

The preservative includes, for example, organic sulfur-based, organic nitrogen sulfur-based, organic halogen-based, haloallylsulfone-based, iodopropargyl-based, N-haloalkylthio-based, nitrile-based, pyridine-based, 8-oxyquinoline-based, benzothiazole-based, isothiazoline-based, dithiol-based, pyridinoxide-based, nitropropane-based, organic tin-based, phenol-based, quaternary ammonium salt-based, triazine-based, thiazine-based, anilide-based, adamantane-based, dithiocarbamate-based, brominated indanon-based, benzyl bromoacetate-based, inorganic salt-based compounds, and the like. Examples of the organic halogen-based compounds include sodium pentachlorophenol and the like. Examples of the pyridine oxide-based compounds include 2-pyridinethiol-1-oxide sodium salt and the like. Examples of the isothiazoline-based compounds include 1,2-benzisothiazolin-3-one, 2-n-octyl-4-isothiazolin-3-one, 5-chloro-2-methyl-4-isothiazolin-3-one, 5-chloro-2-methyl-4-isothiazolin-3-one magnesium chloride, 5-chloro-2-methyl-4-isothiazolin-3-one calcium chloride, 2-methyl-4-isothiazolin-3-one calcium chloride, etc. Examples of other preservative antifungal agents include anhydrous sodium acetate, sodium sorbate, sodium benzoate, Proxel GXL (S) and Proxel XL-2 (S), trade names of Lonza Corporation.

Examples of the surfactant include known surfactants such as anionic, cationic, amphoteric, nonionic, silicone-based, fluorine-based surfactants, etc.

Examples of the anionic surfactant include alkyl sulfonate salts, alkylcarboxylate salts, α-olefin sulfonate salts, polyoxyethylene alkyl ether acetate salts, N-acylamino acid and salts thereof, N-acylmethyltaurine salts, alkylsulfate salt polyoxyalkyl ether sulfate salts, alkylsulfate salt polyoxyethylene alkyl ether phosphate salts, rosin acid soap, castor oil sulfate ester salts, lauryl alcohol sulfate ester salts, alkylphenol-type phosphate esters, alkyl-type phosphate esters, alkylarylsulfonate salts, diethyl sulfosuccinate salts, diethylhexyl sulfosuccinate salts, dioctyl sulfosuccinate salts, and the like. Examples of commercially available products include Hytenol LA-10, LA-12, LA-16, Neohytenol ECL-30S, ECL-45, etc., all of which are manufactured by DKS Co., Ltd.

Examples of the cationic surfactant include 2-vinylpyridine derivatives, poly(4-vinylpyridine) derivatives, etc.

Examples of the amphoteric surfactant include lauryldimethylaminoacetic acid betaine, 2-alkyl-N-carboxymethyl-N-hydroxyethyl imidazolinium betaine, coconut oil fatty acid amide propyldimethylaminoacetic acid betaine, polyoctylpolyaminoethylglycine, imidazoline derivatives and the like.

Examples of the nonionic surfactant include those based on ethers such as polyoxyethylene nonylphenyl ether, polyoxyethylene octylphenyl ether, polyoxyethylene dodecylphenyl ether, polyoxyethylene oleyl ether, polyoxyethylene lauryl ether, polyoxyethylene alkyl ethers, etc.; those based on esters such as polyoxyethylene oleate ester, polyoxyethylene distearate ester, sorbitan laurate, sorbitan monostearate, sorbitan monooleate, sorbitan sesquioleate, polyoxyethylene monooleate, polyoxyethylene stearate, etc.; those based on acetyleneglycols (alcohols) such as 2,4,7,9-tetramethyl-5-decyne-4,7-diol, 3,6-dimethyl-4-octyne-3,6-diol, 3,5-dimethyl-1-hexyn-3-ol, etc.; Surfynol 104, 105, 82, and 465 manufactured by Air Products Japan Co., Ltd. Olfine STG, etc.; those based on polyglycol ethers (e.g., Tergitol 15-S-7, etc. manufactured by SIGMA-ALDRICH).

Examples of the silicone-based surfactant include polyether modified siloxanes, polyether modified polydimethylsiloxanes, and the like. Examples of commercially available products include BYK-347 (polyether-modified siloxane); BYK-345 and BYK-348 (polyether-modified polydimethylsiloxane), all of which are manufactured by Byk-Chemie GmbH., and the like.

Examples of the fluorine-based surfactant include perfluoroalkyl sulfonate compounds, perfluoroalkyl carboxylate-based compounds, perfluoroalkyl phosphate ester compounds, perfluoroalkyl ethylene oxide adducts, polyoxyalkylene ether polymer compounds having a perfluoroalkylether group in their side chains, etc. Examples of commercially available products include Zonyl TBS, FSP, FSA, FSN-100, FSN, FSO-100, FSO, FS-300, Capstone FS-30 and FS-31 (all of which are manufactured by DuPont); PF-151N and PF-154N (both of which are manufactured by Omnova Solutions Inc.); and the like.

As the pH adjuster, any material can be used as long as the pH of a colored dispersion liquid can be controlled to approximately 5 to 11 without adversely affecting the colored dispersion liquid to be prepared. Examples thereof include alkanolamines, such as diethanolamine, triethanolamine, N-methyldiethanolamine, etc.; hydroxides of alkali metals, such as lithium hydroxide, sodium hydroxide, potassium hydroxide, etc.; ammonium hydroxide (ammonia water); carbonate salts of alkali metals, such as lithium carbonate, sodium carbonate, sodium hydrogen carbonate, potassium carbonate, etc.; alkali metal salts of organic acids, such as potassium acetate, etc.; inorganic bases, such as sodium silicate, disodium phosphate, etc.; and the like; and triethanolamine is preferred.

Examples of the chelating reagent include sodium ethylenediaminetetraacetate, sodium nitrilotriacetate, sodium hydroxyethyl ethylenediaminetriacetate, sodium diethylenetriaminepentaacetate, sodium uracil diacetate, and the like.

Examples of the rust inhibitor include acidic sulfite salts, sodium thiosulfate, ammonium thioglucolate, diisopropylammonium nitrite, pentaerythritol tetranitrate, dicyclohexylammonium nitrite, etc.

Examples of the water-soluble ultraviolet absorber include sulfonated benzophenone-based compounds, benzotriazol-based compounds, salicylic acid-based compounds, cinnamic acid-based compounds, triazine-based compounds, etc.

Examples of the water-soluble polymer compound include polyvinyl alcohol, cellulose derivatives, polyamines, polyimines, etc.

Examples of the viscosity modifier include a water-soluble polymer compound in addition to the water-soluble organic solvent, and examples thereof include polyvinyl alcohol, cellulose derivatives, polyamines, polyimines, etc.

Examples of the dye dissolving agent include urea, ε-caprolactam, ethylene carbonate, etc.

As an antioxidant, a variety of organic anti-fading agents and metal complex-based anti-fading agents can be used. Examples of the organic anti-fading agent include hydroquinones, alkoxyphenols, dialkoxyphenols, phenols, anilines, amines, indanes, chromans, alkoxyanilines, heterocyclics, and the like. Examples of the metal complex-based anti-fading agents include nickel complexes, zinc complexes, etc.

Examples of the resin emulsion include emulsions formed of an acrylic resin, an epoxy resin, a urethane resin, a polyether resin, a polyamide resin, an unsaturated polyester resin, a phenol resin, a silicone resin, a fluorine resin, a polyvinyl resin, (vinyl chloride, vinyl acetate, polyvinyl alcohol, etc.) an alkyd resin, a polyester resin, an amino resin (a melanin resin, a urea resin, a melanin formaldehyde resin, etc.), and the like. The resin emulsion may contain two or more types of resins. In addition, the two or more types of resins may form a core/shell structure. Among the resin emulsions, a urethane resin emulsion is preferred.

The urethane resin emulsion is available as a commercial product, and many of them are in the form of an emulsion with a solid content of 30 to 60% by mass. Examples of commercially available products of the urethane resin emulsion include PERMARIN UA-150, 200, 310, 368 and 3945 and UCOAT UX-320 (all of which are manufactured by Sanyo Chemical Industries, Ltd.); HYDRAN WLS-201 and 210 and latex of HW-312B (all of which are manufactured by DIC Corporation); Superflex 150, 170 and 470 (all of which are manufactured by DKS Co., Ltd.); etc. Among these, examples of the polycarbonate-based urethane resin include PERMARIN UA-310 and 3945; UCOAT UX-320; and the like. Further, examples of the polyether-based urethane resin include PERMARIN UA-150 and 200; UCOAT UX-340; and the like.

The urethane resin in the urethane resin emulsion preferably has an SP value of 8 to 24 (cal/cm³)^{1/2}), more preferably 8 to 17 (cal/cm³)^{1/2}), and most preferably 8 to 11 (cal/cm³)^{1/2}). Note that the SP value of the urethane resin is calculated by a Fedors method. When the urethane resin has an acidic group and this acidic group is neutralized to prepare an emulsion, the SP value of the urethane resin before neutralization is used.

When the urethane resin in the urethane resin emulsion has an acidic group such as a carboxy group, a sulfo group, or a hydroxy group, the acidic group may be in the form of an alkali salt. For example, an acidic group can be changed to an alkali salt by charging a urethane resin having an acidic group into water and stirring to prepare an aqueous solution, and then charging an alkaline compound into the aqueous solution to adjust the pH to 6.0 to 12.0. Examples of the alkaline compound include hydroxides of alkali metals such as lithium hydroxide, sodium hydroxide, potassium hydroxide, etc.; hydroxides of alkaline earth metals such as beryllium hydroxide, magnesium hydroxide, calcium hydroxide, strontium hydroxide, etc. One type of the alkaline compound may be used alone or two or more types thereof may be used in combination.

### [Process for preparing colored dispersion liquid, etc.]

As a method for preparing the colored dispersion liquid according to the present embodiment, for example, a method of preparing an aqueous dispersion liquid containing component (A) to component (C) and further adding an additive such as a water-soluble organic solvent, as required, may be mentioned.

Examples of the method of preparing an aqueous dispersion include a known method such as stirring and mixing each component constituting the aqueous dispersion using a sand mill (bead mill), a roll mill, a ball mill, a paint shaker, an ultrasonic disperser, a high-pressure emulsifier, or the like. For example, when a sand mill is used, first, each component and beads as a dispersion medium are charged into a sand mill. As the beads, glass beads having a particle diameter of 0.01 to 1 mm, zirconia beads, or the like can be used. The amount of the beads to be used is preferably 2 to 6 parts by mass per 1 part by mass of the dispersion target. Then, the sand mill is operated to perform a dispersion treatment. Dispersion treatment conditions are generally preferably 1 to 20 hours at 1,000 to 2,000 rpm. Then, the beads are removed by filtration or the like after the dispersion treatment, thereby obtaining an aqueous dispersion.

The thus-prepared colored dispersion liquid may be subjected to microfiltration using a membrane filter or the like. In particular, when the colored dispersion liquid is used as an ink for inkjet textile printing, it is preferable to perform microfiltration for the purpose of preventing clogging or the like of the nozzle. The pore size of the filter used for microfiltration is typically 0.1 to 1 µm, and preferably 0.1 to 0.8 µm.

In addition, the viscosity at 25°C of the colored dispersion liquid as described in the present embodiment is preferably about 3 to 20 mPa·s when measured by an E-type viscometer from the viewpoint of discharge responsiveness at high speed. Further, the surface tension at 25°C of the colored dispersion liquid as described in the present embodiment is about 20 to 55 mN/m when measured by a plate method. Actually, the surface tension at 25°C is adjusted to obtain an appropriate physical property value in consideration of the discharge amount, response speed, ink droplet flight characteristics, etc. of the inkjet printer to be used.

The colored dispersion liquid as described in the present embodiment can be used in various fields, and is suitable for a waterborne writing ink, a waterborne printing ink, an information recording ink, textile printing, and the like. The colored dispersion liquid as described in the present embodiment is particularly preferably used as an ink for inkjet textile printing.

According to the colored dispersion liquid as described in the present embodiment, it is possible to effectively suppress particles in the colored dispersion liquid from aggregating during storage and increasing in average particle diameter, and also to effectively suppress particles from settling during storage. In other words, according to colored dispersion liquid of the present embodiment, it is possible to stably maintain a dispersed state of particles in the colored dispersion liquid.

In addition, the colored dispersion liquid as described in the present embodiment has a good initial filling property to an inkjet printer head and good continuous printing stability. Further, it is possible to obtain a clear image without bleeding of the image on paper after printing.

### <Recording medium>

The recording medium as described in the present embodiment is a recording medium to which the colored dispersion liquid as described in the present embodiment is adhered. The recording medium is not particularly limited as long as the recording medium is recordable with the colored dispersion liquid as described in the preset embodiment, and a fiber, paper (normal paper, inkjet exclusive paper, etc.) can be mentioned. In particular, the recording medium as described in the present embodiment is preferably a hydrophobic fiber to which the colored dispersion liquid as described in the present embodiment is attached.

Examples of the hydrophobic fiber include polyester fibers, nylon fibers, triacetate fibers, diacetate fibers, polyamide fibers, and mixed spun fibers, etc. using two or more types of these fibers. Mixed spun fibers of these hydrophobic fibers with regenerated fibers such as rayon or natural fibers such as cotton, silk, wool, etc. are also included in the hydrophobic fibers in this specification. Among the fibers, those having an ink-receiving layer (anti-bleeding layer) are also known, and such fibers are also included in the hydrophobic fibers. A method for forming the ink-receiving layer is known technology and the fiber having an ink-receiving layer can also be obtained as a commercially available product. The material, structure, and the like of the ink-receiving layer are not particularly limited, and may be used as appropriate depending upon the purpose and the like.

### <Method for textile printing of hydrophobic fibers>

The method for textile printing of a hydrophobic fiber as described in the present embodiment is a method for printing on a hydrophobic fiber using the colored dispersion liquid as described in the present embodiment described above. Methods for printing of a hydrophobic fiber are roughly classified into a direct printing method and a sublimation transfer method.

The direct printing method includes a printing step in which a droplet of the colored dispersion liquid is adhered to a hydrophobic fiber by an inkjet printer to obtain a recorded image such as a letter or a pattern, a fixing step in which the dye in the colored dispersion liquid adhered to the hydrophobic fiber in the printing step is fixed to the hydrophobic fiber by heat, and a washing step of washing the unfixed dye remaining in the hydrophobic fiber.

The fixing step is generally performed by known steaming or baking. Examples of the steaming include a method in which a dye is attached (also referred to as wet heat fixing) to a hydrophobic fiber by treating the hydrophobic fiber with a high-temperature steamer for about 10 minutes at usually 170 to 180°C, or a high-pressure steamer for about 20 minutes at usually 120 to 130°C. Examples of the baking (thermosol) include a method in which a dye is attached (also referred to as dry heat fixation) to a hydrophobic fiber by treating the hydrophobic fiber at 190 to 210°C for about 60 to 120 seconds.

The washing step is a step of washing the obtained fiber with warm water and, if necessary, with ambient temperature water. The warm water or ambient temperature water to be used for washing may contain a surfactant. The washed hydrophobic fiber is also preferably dried, usually at 50 to 120°C for 5 to 30 minutes.

On the other hand, the sublimation transfer method includes printing by adhering a droplet of the colored dispersion liquid to an intermediate recording medium by an inkjet printer to obtain a recorded image, such as a letter, a pattern, etc., and transferring by contacting a hydrophobic fiber with a surface of the intermediate recording medium on which the colored dispersion liquid is adhered to transfer the recorded image to the hydrophobic fiber by heat treatment.

As the intermediate recording medium, it is preferable that the dye in the adhered colored dispersion liquid does not aggregate on its surface and the intermediate recording medium does not interfere with sublimation of the dye when transfer of the recorded image to the hydrophobic fiber is performed. As an example of such an intermediate recording medium, a paper on the surface of which an ink-receiving layer is formed with inorganic fine particles such as silica may be mentioned, and a special paper for inkjet, or the like may be used.

The heat treatment in the transfer step typically includes dry heat treatment at about 190 to 200°C.

The method for textile printing of a hydrophobic fiber as described in the present embodiment may further include a pretreating step of pretreating the hydrophobic fiber for the purpose of preventing bleeding or the like. Examples of this pretreatment step include a step of imparting an aqueous solution (pretreatment liquid) containing a sizing agent, an alkaline substance, a reduction inhibitor, and a hydrotropic agent to a hydrophobic fiber before the colored dispersion liquid is adhered.

Examples of the sizing agent include natural gums such as guar, locust bean, etc.; starches; marine algae such as sodium alginate, Gloiopeltis, etc.; plant skin such as pectic acid, etc.; fibrous derivatives such as methyl fibrin, ethyl fibrin, hydroxyethyl cellulose, carboxymethyl cellulose, etc.; processed starch such as carboxymethyl starch, etc.; synthetic glue such as polyvinyl alcohol and polyacrylate esters; and the like. Sodium alginate is preferable.

The alkaline substance includes, for example, alkali metal salts of inorganic acids or organic acids; salts of alkaline earth metals; compounds which liberate alkali when heated; and the like. Alkali metal hydroxides and alkali metal salts are preferable. Examples include alkali metal hydroxides such as sodium hydroxide, calcium hydroxide, etc.; alkali metal salts of inorganic compounds such as sodium carbonate, sodium bicarbonate, potassium carbonate, sodium dihydrogen phosphate, disodium hydrogen phosphate, sodium phosphate, etc.; alkali metal salts of organic compounds such as sodium formate, sodium trichloroacetate, etc.; and the like. Sodium bicarbonate is preferable.

As the reduction inhibitor, sodium meta-nitrobenzenesulfonate is preferable. As the hydrotropic agent, ureas such as urea, dimethylurea, etc. may be exemplified, and urea is preferable.

With regard to the sizing agent, the alkaline substance, the reduction inhibitor, and the hydrotropic agent, any one type may be used alone, or two or more types may be used in combination.

The mixing ratio of each component in the pretreating liquid is, for example, 0.5 to 5% by mass of the sizing agent, 0.5 to 5% by mass of sodium bicarbonate, 0 to 5% by mass of sodium meta-nitrobenzenesulfonate, 1 to 20% by mass of urea, and the balance of water.

Methods of attaching the pretreating liquid to hydrophobic fibers include, for example, a padding method. The aperture ratio of padding is preferably about 40 to 90%, and more preferably about 60 to 80%.

### EXAMPLES

In the following, the present invention is explained in more detail by way of the Examples; however, the present invention is not limited to these Examples. Unless otherwise indicated in the Examples, "part(s)" and "%" mean part(s) by mass and % by mass, respectively. Note that all of the aqueous dispersion liquids and magenta inks in the Examples are included in the colored dispersion liquids described above.

### <Synthesis Example 1: Synthesis of anthraquinone-based compound H>

Chlorosulfonic acid (28 parts) was cooled to 5°C or less in an ice bath, Kayaset Red B (manufactured by Nippon Kayaku Co., Ltd., C.I. Disperse Red 60) (10 parts) was mixed at 10°C or less, and the mixture was reacted while stirring at 30°C for 20 hours. The resulting reaction solution was poured into iced water to precipitate crystals. The obtained crystals were filtered off, and then washed with pure water to obtain a reddish brown wet cake. Then, n-butylamine (11 parts) was mixed to water (25 parts), and the total amount of the wet cake was added at 30°C. The reaction was carried out under stirring at the same temperature for 20 hours, and the obtained reaction solution was poured into iced water to precipitate crystals. The obtained crystals were filtered off, washed with cold pure water, and then dried at 80°C, thereby obtaining an anthraquinone-based compound H (13 parts) represented by the following formula (a1-1-1).

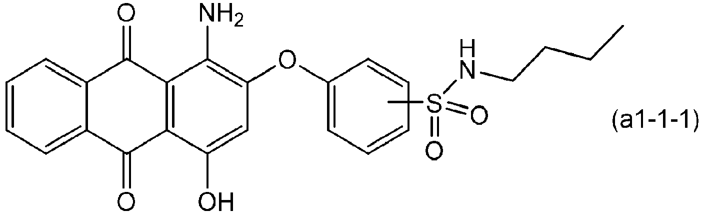

### <Synthesis Example 2: Synthesis of anthraquinone-based compound I>

Chlorosulfonic acid (28 parts) was cooled to 5°C or less in an ice bath, Kayaset Red B (manufactured by Nippon Kayaku Co., Ltd., C.I. Disperse Red 60) (10 parts) was mixed at 10°C or less, and the mixture was reacted while stirring at 30°C for 20 hours. The resulting reaction solution was poured into iced water to precipitate crystals. The obtained crystals were filtered off, and then washed with pure water to obtain a reddish brown wet cake. Then, water (30 parts) and a 25% ammonia water (30 parts) were mixed, and the total amount of the wet cake was added at 30°C. The reaction was carried out under stirring at the same temperature for 20 hours, and the obtained reaction solution was poured into iced water to precipitate crystals. The obtained crystals were filtered off, washed with cold pure water, and then dried at 80°C, thereby obtaining an anthraquinone-based compound I (11.4 parts) represented by the following formula (a1-1-2).

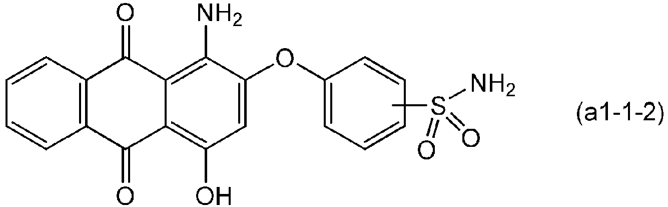

### <Examples 1 to 9: Preparation of aqueous dispersion liquids 1 to 9>

Glass beads with a diameter of 0.2 mm were added to mixtures each obtained by mixing the respective components to be added first described in Tables 1 to 2 below, and the mixtures were subjected to a dispersion treatment in a sand mill for about 15 hours under water cooling. To the obtained liquids, components to be added later described in Tables 1 to 2 were added to adjust dye contents to 15%, and then the obtained mixtures were filtered through a glass fiber filter paper GC-50 (pore diameter of the filter: 0.5 µm manufactured by Advantec Ltd.), thereby obtaining aqueous dispersion liquids 1 to 9.

### <Comparative Examples 1 to 4: Preparation of aqueous dispersions 10 to 13>

Glass beads with a diameter of 0.2 mm were added to mixtures each obtained by mixing the respective components to be added first described in Table 3, and then the mixtures were subjected to a dispersion treatment in a sand mill for about 15 hours under water cooling. To the obtained liquids, components to be added later described in Table 3 were added to adjust the dye contents to 15%, and then filtered through a glass fiber filter paper GC-50 (ADVANTEC Co., Ltd., pore diameter of the filter: 0.5 µm) to obtain aqueous dispersions 10 to 13.

**[Table 1]**

| | | Example 1 | Example 2 | Example 3 | Example 4 | Example 5 | Example 6 |
|---|---|---|---|---|---|---|---|
| | | Aqueous dispersion liquid 1 | Aqueous dispersion liquid 2 | Aqueous dispersion liquid 3 | Aqueous dispersion liquid 4 | Aqueous dispersion liquid 5 | Aqueous dispersion liquid 6 |
| Componen ts to be added first | DR92 (A) | 0.1 | 0.2 | 0.4 | 0.6 | 1 | 6 |
| | DR60 (B) | 20 | 20 | 20 | 20 | 20 | 20 |
| | TS-2000 (C) | 6 | 6 | 6 | 6 | 6 | 6 |
| | SM-57 (C) | 8.6 | 8.6 | 8.6 | 8.6 | 8.6 | 8.6 |
| | Proxel GXL | 0.13 | 0.13 | 0.13 | 0.13 | 0.13 | 0.13 |
| | Surfynol 104PG50 | 0.27 | 0.27 | 0.27 | 0.27 | 0.27 | 0.27 |
| | Ion-exchanged water | 53.8 | 53.7 | 53.5 | 53.3 | 52.9 | 47.9 |
| Componen ts to be added later | Ion-exchanged water | 44.4 | 44.4 | 44.4 | 44.4 | 44.4 | 44.4 |
| (B)/(A) | | 200 | 100 | 50 | 33.33 | 20 | 3.33 |

**[Table 2]**

| | | Example 7 | Example 8 | Example 9 |
|---|---|---|---|---|
| | | Aqueous dispersion liquid 7 | Aqueous dispersion liquid 8 | Aqueous dispersion liquid 9 |
| Components to be added first | Anthraquinone-based compound H (A) | 0.4 | | |
| | Anthraquinone-based compound I (A) | | 0.4 | |
| | DR146 (A) | | | 0.4 |
| | DR60 (B) | 20 | 20 | 20 |
| | TS-2000 (C) | 6 | 6 | 6 |
| | SM-57 (C) | 8.6 | 8.6 | 8.6 |
| | Proxel GXL | 0.13 | 0.13 | 0.13 |
| | Surfynol 104PG50 | 0.27 | 0.27 | 0.27 |
| | Ion-exchanged water | 53.5 | 53.5 | 53.5 |
| Components to be added later | Ion-exchanged water | 44.4 | 44.4 | 44.4 |
| (B)/(A) | | 50 | 50 | 50 |

**[Table 3]**

| | | Comparative Example 1 | Comparative Example 2 | Comparative Example 3 | Comparative Example 4 |
|---|---|---|---|---|---|
| | | Aqueous dispersion liquid 10 | Aqueous dispersion liquid 11 | Aqueous dispersion liquid 12 | Aqueous dispersion liquid 13 |
| Components to be added first | DR92 (A) | | | 0.05 | 6.5 |
| | Anthraquinone-based compound A | | 0.4 | | |
| | DR60 (B) | 20 | 20 | 20 | 20 |
| | TS-2000 (C) | 6 | 6 | 6 | 6 |
| | SM-57 (C) | 8.6 | 8.6 | 8.6 | 8.6 |
| | Proxel GXL | 0.13 | 0.13 | 0.13 | 0.13 |
| | Surfynol 104PG50 | 0.27 | 0.27 | 0.27 | 0.27 |
| | Ion-exchanged water | 53.9 | 53.5 | 53.85 | 47.4 |
| Components to be added later | Ion-exchanged water | 44.4 | 44.4 | 44.4 | 44.4 |
| (B)/(A) | | - | - | 400 | 3.08 |

In Tables 1 to 3, notations (A), (B), and (C) after the end of the component names indicate that the components are component (A), component (B), and component (C), respectively. Further, in Tables 1 to 3, numerical values of the respective components refer to the number of parts added. Furthermore, in Tables 1 to 3, values indicated to the second decimal places as the values of ratios (B)/(A) are values obtained by rounding up the third decimal places.

Abbreviations and the like in Tables 1 to 3 have the following meanings.
DR92: C.I. Disperse Red 92
DR146: C.I. Disperse Red 146
Anthraquinone-based compound A: Compound described as a dye derivative b in the PCT International Publication No. WO2017/038747
DR60: C.I. Disperse Red 60
TS-2000: Polyoxyethylene styrylphenyl ether (manufactured by Toho Chemical Industry Co., Ltd.)
SM-57: Polyoxyethylene styrylphenyl ether sulfate (manufactured by Toho Chemical Industry Co., Ltd.)
Proxel GXL: preservative antifungal agent (manufactured by Lonza)
Surfynol 104PG50: Surfynol 104 (acetylene glycol surfactant, manufactured by Air Products Japan Co., Ltd.) diluted with propylene glycol to 50% concentration

### <Examples 10 to 18: Preparation of magenta inks 1 to 9>

Aqueous dispersion liquids 1 to 9 obtained in Examples 1 to 9 and the respective components described in Tables 4 to 5 below were mixed and stirred for 30 minutes, and then each of the resulting mixtures was filtered through a glass fiber filter paper GC-50 (manufactured by Advantec Ltd., pore diameter of the filter: 0.5 µm) to obtain magenta inks 1 to 9.

### <Comparative Examples 5 to 8: Preparation of magenta inks 10 to 13>

Aqueous dispersion liquids 10 to 13 obtained in Comparative Examples 1 to 4 and each of the components described in Table 6 below were mixed and stirred for 30 minutes, and then filtered through a glass fiber filter paper GC-50 (manufactured by Advantec Ltd., pore diameter: 0.5 µm) to prepare magenta inks 10 to 13.

**[Table 4]**

| | | Example 10 | Example 11 | Example 12 | Example 13 | Example 14 | Example 15 |
|---|---|---|---|---|---|---|---|
| | | Magenta ink 1 | Magenta ink 2 | Magenta ink 3 | Magenta ink 4 | Magenta ink 5 | Magenta ink 6 |
| Aqueous dispersion liquid 1 | | 50 | | | | | |
| Aqueous dispersion liquid 2 | | | 50 | | | | |
| Aqueous dispersion liquid 3 | | | | 50 | | | |
| Aqueous dispersion liquid 4 | | | | | 50 | | |
| Aqueous dispersion liquid 5 | | | | | | 50 | |
| Aqueous dispersion liquid 6 | | | | | | | 50 |
| Other compone nts | Glycerin | 15 | 15 | 15 | 15 | 15 | 15 |
| | Propylene glycol | 10 | 10 | 10 | 10 | 10 | 10 |
| | Methyl triglycol | 5 | 5 | 5 | 5 | 5 | 5 |
| | BYK-348 | 0.8 | 0.8 | 0.8 | 0.8 | 0.8 | 0.8 |
| | TEA-80 | 0.1 | 0.1 | 0.1 | 0.1 | 0.1 | 0.1 |
| | Proxel GXL | 0.1 | 0.1 | 0.1 | 0.1 | 0.1 | 0.1 |
| | Ion-exchanged water | Balance | Balance | Balance | Balance | Balance | Balance |
| Total | | 100 | 100 | 100 | 100 | 100 | 100 |

**[Table 5]**

| | | Example 16 | Example 17 | Example 18 |
|---|---|---|---|---|
| | | Magenta ink 7 | Magenta ink 8 | Magenta ink 9 |
| Aqueous dispersion liquid 7 | | 50 | | |
| Aqueous dispersion liquid 8 | | | 50 | |
| Aqueous dispersion liquid 9 | | | | 50 |
| Other components | Glycerin | 15 | 15 | 15 |
| | Propylene glycol | 10 | 10 | 10 |
| | Methyl triglycol | 5 | 5 | 5 |
| | BYK-348 | 0.8 | 0.8 | 0.8 |
| | TEA-80 | 0.1 | 0.1 | 0.1 |
| | Proxel GXL | 0.1 | 0.1 | 0.1 |
| | Ion-exchanged water | Balance | Balance | Balance |
| Total | | 100 | 100 | 100 |

**[Table 6]**

| | | Comparative Example 5 | Comparative Example 6 | Comparative Example 7 | Comparative Example 8 |
|---|---|---|---|---|---|
| | | Magenta ink 10 | Magenta ink 11 | Magenta ink 12 | Magenta ink 13 |
| Aqueous dispersion liquid 10 | | 50 | | | |
| Aqueous dispersion liquid 11 | | | 50 | | |
| Aqueous dispersion liquid 12 | | | | 50 | |
| Aqueous dispersion liquid 13 | | | | | 50 |
| Other components | Glycerin | 15 | 15 | 15 | 15 |
| | Propylene glycol | 10 | 10 | 10 | 10 |
| | Methyl triglycol | 5 | 5 | 5 | 5 |
| | BYK-348 | 0.8 | 0.8 | 0.8 | 0.8 |
| | TEA-80 | 0.1 | 0.1 | 0.1 | 0.1 |
| | Proxel GXL | 0.1 | 0.1 | 0.1 | 0.1 |
| | Ion-exchanged water | Balance | Balance | Balance | Balance |
| Total | | 100 | 100 | 100 | 100 |

In Tables 4 to 6, numerical values of the respective components indicate the number of parts added. Abbreviations and the like in Tables 4 to 6 have the following meanings. BYK-348: Polyether-modified polydimethylsiloxane (manufactured by Byk-Chemie GmbH)
TEA-80: Triethanolamine (manufactured by Oxalis Chemicals Ltd.)
Proxel GXL: Preservative antifunagal agent (manufactured by Lonza)

### <Evaluation>

Using each ink prepared as described above, the following evaluation tests were performed. Results are indicated in Tables 7 to 9 below.

### [Viscosity change test]

With respect to each of the magenta inks, the viscosity at initial stage and the viscosity after storage for three days at 70°C were measured using an E-type viscometer (TV-200, manufactured by Toki Sangyo Co., Ltd.) calibrated by a standard solution JS10 for viscometer calibration (manufactured by Nippon Grease Co., Ltd.) under condition of 25°C, at a rotational speed of 50 rpm. The viscosity change rate was calculated from the viscosity at the initial stage and the viscosity after the storage, and evaluated based on the following criteria. A or B represents a good evaluation, and C represents a poor evaluation.

### -Evaluation criteria-

A: The absolute value of change rate is less than 5%.
B: The absolute value of change rate is 5% or more and less than 15%.
C: The absolute value of change rate is 15% or more.

### [Particle size change test]

With respect to each of the magenta inks, the median diameter (D50, number average particle diameter) at initial stage and the median diameter after storage for three days at 70°C were measured using a MICROTRAC UPA EX150 (manufactured by Microtrac BEL Corp.). The particle diameter change rate was calculated from the particle diameter at the initial stage and the particle diameter after the storage, and evaluated based on the following criteria. A or B represents a good evaluation and C or D represents a poor evaluation

### -Evaluation criteria-

A: The absolute change rate is less than 10%.
B: The absolute change rate is 10% or more and less than 20%.
C: The absolute change rate is 20% or more and less than 50%.
D: The change rate is 50% or more.

### [Confirmation of sedimentation property]

With respect to each of the magenta inks stored for three days at 70°C, the presence or absence of sedimentation was visually confirmed and evaluated based on the following criteria. A or B represents a good evaluation, and C represents a poor evaluation.

### -Evaluation criteria-

A: No sedimentation is confirmed.
B: Slight sedimentation is confirmed.
C: The sedimentation is confirmed to a considerable extent.

### [Confirmation of filterability]

10 mL of each of the magenta inks stored for three days at 70°C was filtered through a filter having a pore size of 0.8 µm (manufactured by ADVANTEC Co., Ltd., DISMIC) and evaluated based on the following criteria. A or B represents a good evaluation, and C represents a poor evaluation.

### -Evaluation criteria-

A: All of the 10 mL of ink can pass through and there is little filtration resistance.
B: All of the 10 mL of ink can pass, but there is filtration resistance.
C: Filter clogging occurs and all of the 10 mL of ink cannot pass through.

**[Table 7]**

| | | Example 10 | Example 11 | Example 12 | Example 13 | Example 14 | Example 15 |
|---|---|---|---|---|---|---|---|
| Viscosity change test | Change rate [%] | 9 | 6 | 3 | 5 | 6 | 14 |
| | Evaluation | B | B | A | B | B | B |
| Particle size change test | Change rate [%] | 8 | 2 | 7 | 9 | 13 | 17 |
| | Evaluation | A | A | A | A | B | B |
| Sedimentation property | Evaluation | A | A | A | A | B | B |
| Filterability | Evaluation | A | A | A | B | B | B |

**[Table 8]**

| | | Example 16 | Example 17 | Example 18 |
|---|---|---|---|---|
| Viscosity change test | Change rate [%] | 7 | 7 | 6 |
| | Evaluation | B | B | B |
| Particle size change test | Change rate [%] | 9 | 8 | 9 |
| | Evaluation | A | A | A |
| Sedimentation property | Evaluation | A | A | A |
| Filterability | Evaluation | A | A | A |

**[Table 9]**

| | | Comparative Example 5 | Comparative Example 6 | Comparative Example 7 | Comparative Example 8 |
|---|---|---|---|---|---|
| Viscosity change test | Change rate [%] | 6 | 3 | -11 | 37 |
| | Evaluation | B | A | B | C |
| Particle size change test | Change rate [%] | 50 | 13 | 21 | 18 |
| | Evaluation | D | B | C | B |
| Sedimentation property | Evaluation | C | C | B | B |
| Filterability | Evaluation | B | B | B | B |

As is apparent from the results of Tables 7 to 9, all of the magenta inks of Examples 10 to 18 prepared using the aqueous dispersions of Examples 1 to 9 showed no significant change in viscosity or particle diameter after the storage at high temperature, or no sedimentation, exhibiting excellent storage stability. On the other hand, the magenta inks of Comparative Examples 5 to 8 prepared using the aqueous dispersions of Comparative Examples 1 to 4 were inferior in storage stability to the magenta inks of Examples 10 to 18.

## Claims

1. A colored dispersion liquid, comprising: (A) a dye derivative, (B) a water-insoluble dye, (C) a dispersant, and water,
wherein the dye derivative (A) comprises an anthraquinone-based compound represented by the following formula (a1): 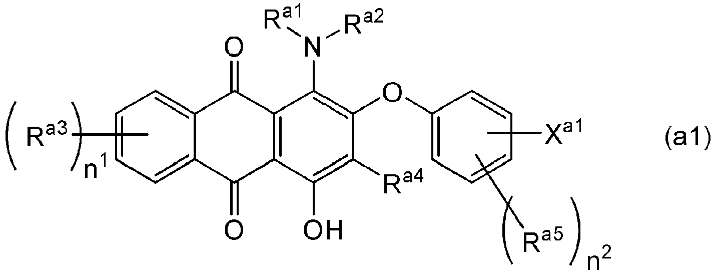
wherein R^{a1} to R^{a5} each independently represent a hydrogen atom, a C1 to C4 alkyl group which may have a substituent, a cyclic alkyl group which may have a substituent, an aryl group which may have a substituent, or a halogen atom; n¹ and n² each independently represent an integer of 1 to 4, when n¹ represents an integer of 2 to 4, R^{a3} in the number of n¹ may be the same as or different from each other, when n² represents an integer of 2 to 4, R^{a5} in the number of n² may be the same as or different from each other, and X^{a1} represents a group represented by any one of the following formulas (a2) to (a5): 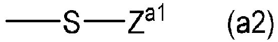 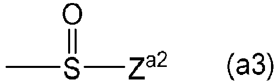 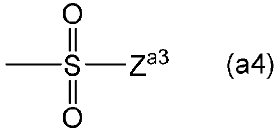 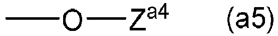
wherein Z^{a1} represents an aliphatic hydrocarbon group which may have a substituent or an aromatic hydrocarbon group which may have a substituent, Z^{a2} represents an amino group which may have a substituent or an alkoxy group which may have a substituent, Z^{a3} represents an amino group which may have a substituent, an aliphatic hydrocarbon group which may have a substituent, an aromatic hydrocarbon group which may have a substituent, an alkoxy group which may have a substituent, an aryloxy group which may have a substituent, a pyridinooxy group, a hydroxy group, or a halogen atom, and Z^{a4} represents a C1 to C4 alkyl group,
wherein the dye derivative (A) does not simultaneously comprise a compound represented by the formula (a1) wherein X^{a1} represents a group represented by the formula (a4) and a compound represented by the formula (a1) wherein X^{a1} represents a group represented by the formula (a5),
wherein the dispersant (C) comprises a polyoxyethylene arylphenyl ether and a polyoxyethylene arylphenyl ether sulfate, and
wherein a mass ratio ((B)/(A)) of the water-insoluble dye (B) to the dye derivative (A) satisfies a relation of 400 > (B)/(A) > 3.125.

2. The colored dispersion liquid according to claim 1, wherein the mass ratio ((B)/(A)) of the water-insoluble dye (B) to the dye derivative (A) satisfies a relation of 400 > (B)/(A) > 19.

3. The colored dispersion liquid according to claim 1 or 2, wherein the anthraquinone-based compound represented by the formula (a1) comprises a compound represented by the following formula (al-1): 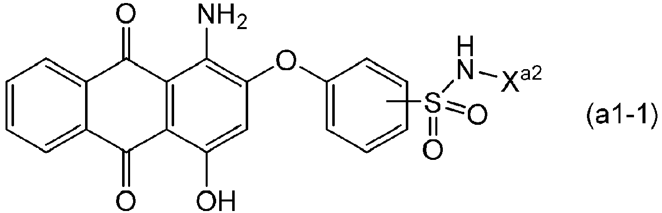 wherein X^{a2} represents a hydrogen atom, a phenyl group, a n-butyl group, or a 3-ethoxypropyl group.

4. The colored dispersion liquid according to any one of claims 1 to 3, wherein the water-insoluble dye (B) is a disperse dye.

5. The colored dispersion liquid according to claim 4, wherein the disperse dye is a disperse dye having an anthraquinone skeleton.

6. The colored dispersion liquid according to claim 5, wherein the disperse dye having an anthraquinone skeleton is a disperse dye represented by the following formula (b1): 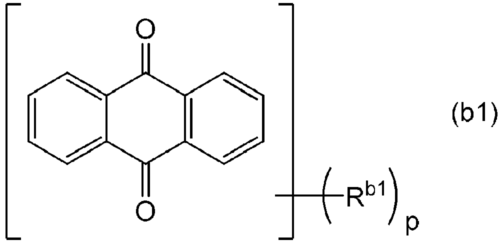 wherein R^{b1} represents a hydrogen atom or substituent, p represents an integer of 0 to 6, and when p represents an integer of 2 to 6, R^{b1} in the number of p may be the same as or different from each other.

7. The colored dispersion liquid according to claim 6, wherein the disperse dye represented by the formula (b1) is C.I. Disperse Red 60.

8. A recording medium, comprising the colored dispersion liquid according to any one of claims 1 to 7 adhered thereto.

9. The recording medium according to claim 8, wherein the recording medium is a hydrophobic fiber.

10. A method for textile printing of a hydrophobic fiber, comprising:
printing by adhering a droplet of the colored dispersion liquid according to any one of claims 1 to 7 to an intermediate recording medium to obtain a recorded image, and
transferring the recorded image to the hydrophobic fiber by contacting a hydrophobic fiber with a surface of the intermediate recording medium on which the colored dispersion liquid is adhered, followed by heat treatment.
